# EUROPEAN PATENT APPLICATION

(11) **EP 4 159 197 A1**
(43) Date of publication of application: **05.04.2023**
(21) Application number: 21814359.2
(22) Date of filing: 28.05.2021
(51) Int. Cl.: A61K 9/08, A61K 9/107, A61K 31/05, A61K 31/661, A61K 47/06, A61K 47/10, A61K 47/12, A61P 23/00, A61P 25/06, A61P 25/08, A61P 25/18, A61P 25/20, A61P 25/22, A61P 25/24

(54) **PHARMACEUTICAL PREPARATION AND PREPARATION METHOD THEREFOR**

(30) Priority: 29.05.2020 CN 202010472446
(71) Applicant: Sichuan Haisco Pharmaceutical Co., Ltd., Sichuan 611130 (CN); Liaoning Haisco Pharmaceutical Co., Ltd., Xingcheng City Huludao Liaoning 125107 (CN); Haisco Pharmaceutical Group Co., Ltd., Tibet 856000 (CN)
(72) Inventor: MO, Yi, Chengdu, Sichuan 611130 (CN); LI, Honghu, Chengdu, Sichuan 611130 (CN); ZHANG, Yuquan, Chengdu, Sichuan 611130 (CN); TANG, Yu, Chengdu, Sichuan 611130 (CN)
(74) Representative: Teuten, Andrew John
(86) International application number: PCT/CN2021/096890
(87) International publication number: WO 2021/239130

(57) **Abstract**

A pharmaceutical preparation containing a GABA_{A} receptor reinforcing agent and a bacteriostat, a preparation method therefor, and use of the pharmaceutical preparation in preparing a drug for inducing and maintaining anesthesia in animals or humans, promoting sedation and hypnosis in animals or humans, treating and/or preventing anxiety, depression, insomnia, nausea, vomiting, migraine, schizophrenia, convulsions, and epilepsy. The GABA_{A} receptor reinforcing agent is as represented by formula (I) or a stereoisomer, a pharmaceutically acceptable salt or a prodrug thereof, wherein R¹, R² and n are defined in the description.

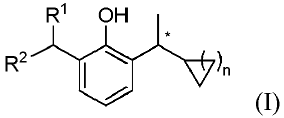

## Description

### Technical Field

The present invention relates to a pharmaceutical preparation of a GABA_{A} receptor reinforcing agent and a preparation method therefor. The GABA_{A} receptor reinforcing agent is a compound (I) or a pharmaceutically acceptable salt or a prodrug thereof. The present invention belongs to the technical field of biological medicine.

### Background Art

A drug-loaded fat emulsion contains a large amount of lipid components, which is beneficial to bacterial reproduction. During clinical use, when a syringe extracts a liquid medicine from a sterile container or some accidental non-sterile operations may possibly lead to microbial contamination of medicines, a risk of microbial contamination is brought.

CN201580001777 discloses a preparation method of a pharmaceutical preparation composition containing a GABAA receptor reinforcing agent. The disclosed composition contains a pharmaceutical preparation of a compound of formula (I) as shown below. A fat emulsion mentioned in the preparation method does not contain a bacteriostat,

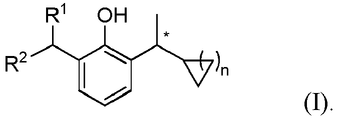

Therefore, there is a need for a pharmaceutical preparation containing the compound (I) which is insensitive to bacterial reproduction under unintentional contamination.

### Summary of the Invention

The present invention provides a stable and safe pharmaceutical preparation containing a GABAA receptor reinforcing agent. The pharmaceutical preparation of the present invention is insensitive to bacterial reproduction under unintentional contamination and provides additional stability.

The pharmaceutical preparation of the present invention contains 0.01 w/v%-5 w/v% of an active ingredient and 0.005 w/v %-0.1 w/v % of a bacteriostat, wherein the active ingredient is a compound of general formula (I) or a stereoisomer, a pharmaceutically acceptable salt or a prodrug thereof, 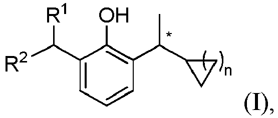 wherein R¹ and R² are each independently selected from H, C₁₋₄ alkyl or C₃₋₆ cycloalkyl; or R¹ and R² together with the carbon atom to which they are attached form C₃₋₆ cycloalkyl; and n is selected from 1 or 2.

The prodrug of the compound of formula (I) is selected from a compound of formula (II) or a stereoisomer and a pharmaceutically acceptable salt thereof: 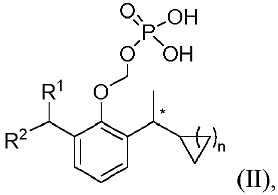 and the pharmaceutically acceptable salt is a salt formed by combining the compound of formula (II) with a metal cation, such as a sodium salt and a potassium salt.

In some embodiments, R¹ is selected from H, methyl, ethyl or isopropyl; and R² is selected from methyl, ethyl, isopropyl or cyclopropyl.

In some embodiments, the compound of general formula (I) is selected from one of the following structures:

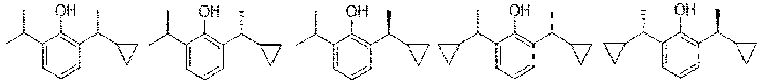

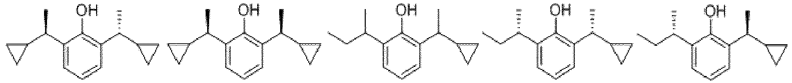

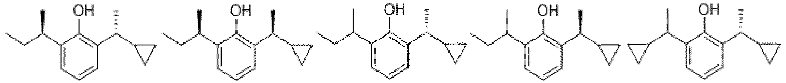

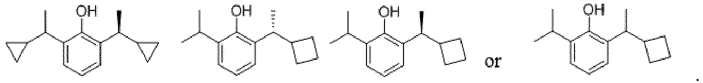

In some embodiments, the compound of general formula (I) is selected from one of the following structures:

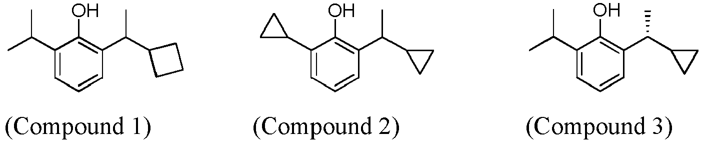

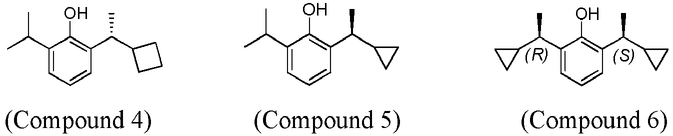

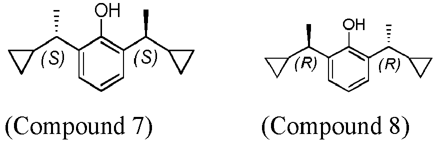

With regard to a preferred solution of the pharmaceutical preparation of the present invention, the pharmaceutical preparation is a fat emulsion comprising:
(1) a compound of general formula (I) or a stereoisomer, a pharmaceutically acceptable salt or a prodrug thereof, in a content of 0.01 w/v%-5 w/v%; preferably 0.05 w/v%-2.5 w/v%; and further preferably 0.1 w/v%-2 w/v%;
(2) an oil ingredient in a content of 5 w/v%-30 w/v%; preferably 5 w/v%-25 w/v%; and further preferably 10 w/v%-25 w/v%;
(3) an emulsifier in a content of 0.1 w/v%-5 w/v%; preferably 0.2 w/v%-3 w/v%; and further preferably 0.2 w/v%-2 w/v%;
(4) an osmotic pressure regulator in a content of 0 w/v%-10 w/v%; preferably 0 w/v%-5 w/v%; and further preferably 0-4 w/v%; and
(5) a bacteriostat in a content of 0.0005 w/v%-0.1 w/v%; preferably 0.001%-0.05%; and further preferably 0.001 w/v%-0.03 w/v%.

In some embodiments, the oil ingredient may be selected from any one of or a mixture of some, at any ratio, of soybean oil, olive oil, fish oil, linseed oil, medium chain triglycerides, and structured triglycerides; preferably selected from any one of or a mixture of some, at any ratio, of soybean oil, olive oil, medium chain triglycerides, and structured triglycerides; more preferably selected from any one of or a mixture of some, at any ratio, of soybean oil, medium chain triglycerides, and structured triglycerides; and further preferably selected from any one of or a mixture of two, at any ratio, of soybean oil and medium chain triglycerides.

The structured triglycerides are prepared by changing the composition or position distribution of fatty acids on a glycerol skeleton by a certain means using a chemical or biological enzymatic method, and have specific molecular structures and functions.

A medium chain triglyceride, also referred to as medium chain fatty acid triglyceride, is composed of 1 molecule of glycerol and 3 molecules of medium chain fatty acids, wherein the number of carbon atoms of the carbon chain of the medium chain fatty acids is 6-10 and the representative main components are octanoic acid (C8, 8 carbon atoms) and decanoic acid (C10, 10 carbon atoms).

In some embodiments, the emulsifier ingredient is selected from any one of or a mixture of some, at any ratio, of poloxamer, Tween-80, polyethylene glycol 15 hydroxystearate, polyoxyethylene 35 castor oil, polyoxyethylene 40 hydrogenated castor oil, egg yolk lecithin or soybean lecithin; preferably selected from any one of or a mixture of some, at any ratio, of poloxamer, egg yolk lecithin or soybean lecithin; and more preferably selected from any one of or a mixture of two, at any ratio, of soybean lecithin or egg yolk lecithin.

In some embodiments, the bacteriostat is selected from any one of or a mixture of some, at any ratio, of sodium caprylate, benzoic acid, benzyl alcohol, sodium benzoate, methyl benzoate, sodium pyrosulfite, sodium sulfite, sodium thiosulfate, pyrogallate, ethylenediaminetetraacetic acid or an ethylenediaminetetraacetic acid salt (such as disodium edetate, calcium edetate, sodium calcium edetate, etc.); and more preferably selected from any one of or a mixture of some, at any ratio, of benzyl alcohol, sodium benzoate, sodium pyrosulfite, ethylenediaminetetraacetic acid or an ethylenediaminetetraacetic acid salt (such as disodium edetate, calcium edetate, sodium calcium edetate, etc.), further preferably selected from any one of or a mixture of some, at any ratio, of ethylenediaminetetraacetic acid, disodium edetate or sodium calcium edetate.

In some embodiments, the osmotic pressure regulator is selected from any one or a mixture of some, at any ratio, of glycerol, saccharides or sugar alcohols, preferably any one or a mixture of some, at any ratio, of glycerol, glucose, fructose, maltose, polyethylene glycol, sorbitol, propylene glycol, xylitol or mannitol, preferably any one or a mixture of some, at any ratio, of glycerol, polyethylene glycol or mannitol, and further preferably glycerol.

In addition to an active agent of a compound of formula (I), an oil ingredient, an emulsifier, a bacteriostat, and an osmotic pressure regulator, the pharmaceutical preparation of the present invention can further contain a pH regulator. The pH regulator is selected from any one or several of sodium hydroxide, potassium hydroxide, triethanolamine, hydrochloric acid, phosphoric acid, citric acid, acetic acid or malic acid, preferably any one or a mixture of some, at any ratio, of sodium hydroxide, potassium hydroxide, triethanolamine, phosphoric acid, citric acid or hydrochloric acid, and further preferably any one or a mixture of some, at any ratio, of sodium hydroxide or hydrochloric acid. The pharmaceutical preparation has a pH value of 3.0-10.0, preferably 4.0-9.0, further preferably 6.0-9.0.

In addition to an active agent of a compound of formula (I), an oil ingredient, an emulsifier, a bacteriostat, an osmotic pressure regulator, and a pH regulator, the pharmaceutical preparation of the present invention can further contain a stabilizer for improving the stability of a fat emulsion and a common surfactant, etc., can also be used as the stabilizer. The stabilizer has a content in a range of 0-2% (w/v) based on the fat emulsion preparation of the present invention. For example, oleic acid and sodium oleate are commonly used as stabilizers for a fat emulsion injection currently, but the stabilizers are not limited thereto.

With regard to a preferred solution of the pharmaceutical preparation of the present invention, the fat emulsion comprises:
(1) an active ingredient of formula (I) in a content of 0.1 w/v%-2 w/v%;
(2) any one of or a mixture of two, at any ratio, of soybean oil or medium chain triglycerides in a content of 10 w/v%-20 w/v% of;
(3) egg yolk lecithin in a content of 0.2 w/v%-2 w/v%;
(4) any one of or a mixture of two, at any ratio, of disodium edetate or sodium calcium edetate or sodium pyrosulfite or benzyl alcohol in a content of 0.001 w/v%-0.03 w/v%;
(5) glycerol in a content of 1 w/v%-4 w/v%; and
(6) any one of or a mixture of two, at any ratio, of oleic acid or sodium oleate in a content of 0.01 w/v%-2 w/v%.

In a preferred solution of the pharmaceutical preparation of the present invention, the fat emulsion has a pH value of 3.0-10.0, preferably 4.0-9.0, further preferably 6.0-9.0.

A method for preparing the pharmaceutical preparation of the present invention comprises the following steps:
(1) preparing an oil phase: in an inert gas environment, adding an emulsifier and a compound of formula (I) into an oil component, and uniformly stirring same as an oil phase;
(2) preparing an aqueous phase: in an inert gas environment, adding an osmotic pressure regulator, a stabilizer, and a bacteriostat into an appropriate amount of water for injection, and uniformly stirring same as an aqueous phase; and
(3) preparing a preparation: in an inert gas environment, slowing adding the oil phase into the aqueous phase under stirring to obtain a primary emulsion; and repeatedly homogenizing the emulsion by a high-pressure homogenizer until emulsion particles are accepted, followed by filtration, filling, sterilization, and cooling to obtain the pharmaceutical preparation of the present invention.

The pH value of the pharmaceutical preparation of the present invention can generally be adjusted to 6.0-9.0. In the preparation method, the stirring method, rotating speed, and time are controlled according to needs. A high shear mixing emulsifier is preferred during the preparation of a primary emulsion and can be selected according to needs. During the homogenization with a high-pressure homogenizer, the homogenizing conditions and time are known to a person skilled in the art, as long as the average particle size of the homogenized emulsion particles is less than or equal to 300 nm and 95% of the particles have a particle size of less than or equal to 1.5 µm. The sterilization can be performed by high-pressure steam sterilization, hot water immersion sterilization, spraying sterilization, etc. As a more preferred example of a sterilization process, high-pressure steam sterilization can be used (for example, 121°C, 12 min).

In the preparation method of the present invention, the inert gas is selected from but not limited to nitrogen.

The preparation method used in the present invention is characterized by uniformly dispersing the compound in general formula (I) in an oil ingredient and an emulsifier and wrapping same by the oil phase, and then adding an aqueous phase containing a bacteriostat. The prepared oil-in-water fat emulsion has good stability and good clinical safety. Accelerated and long-term stability tests prove that the product has a stable quality, facilitates large-scale production, and ensures safety in clinical medication.

The pharmaceutical preparation obtained in the present invention can significantly reduce a microbial contamination of the drug caused by some accidental non-sterile operations and improve the safety of clinical use.

The present invention further provides the use of the pharmaceutical preparation of the present invention in preparing a drug for inducing and maintaining anesthesia in animals or humans, promoting sedation and hypnosis in animals or humans, treating and/or preventing anxiety, depression, insomnia, nausea, vomiting, migraine, schizophrenia, convulsions, and epilepsy.

Unless stated to the contrary, the terms used in the description and claims have the following meanings.

The "w/v" refers to the weight/volume percentage content, i.e., "weight of each component (g)/volume of the solution prior to dispensing (ml)".

### Brief Description of the Drawings

Fig. 1 shows growth curves of 5 test microorganisms in a sample prepared in Example 3; and
Fig. 2 shows growth curves of 5 test microorganisms in a sample prepared in Example 4.

### Detailed Description of Embodiments

The implementation process and beneficial effects of the present invention are described in detail below through specific examples, which are intended to help a person skilled in the art better understand the essence and characteristics of the present invention, and are not intended to limit the scope of implementation of the present application.

The compounds required by the present invention are all prepared according to the method disclosed in patent application CN201580001777.

### Example 1

A formulation is as follows:

| | |
|---|---|
| Compound 1 | 1 g |
| Soybean oil (for injection) | 50 g |
| Medium chain triglyceride | 50 g |
| Egg yolk lecithin | 12 g |
| Glycerol (for injection) | 22.5 g |
| Oleic acid | 0.4 g |
| Edetate disodium | 0.05 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), oleic acid (source: Lipoid GmbH, Germany), and compound 1 were added under high-speed stirring, the materials were uniformly stirred, with the temperature controlled to be 55-75°C, to obtain an oil phase. Disodium edetate (source: Hunan ER-KANG Pharmaceutical Co., Ltd) and glycerol (for injection) [source: Croda Sipo (Sichuan) Co., Ltd)] were added into an appropriate amount of water for injection, mixed, and adjusted to a pH value of 10.10 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 1 just after same was checked to be accepted.

### Example 2

A formulation is as follows:

| | |
|---|---|
| Compound 2 | 5 g |
| Soybean oil (for injection) | 100 g |
| Egg yolk lecithin | 12 g |
| Glycerol (for injection) | 22.5 g |
| Sodium oleate | 0.3 g |
| Sodium calcium edetate | 0.05 g |
| Sodium hydroxide | Q.s. |
| Water for injection, making up the volume to | 1,000 ml |

Soybean oil (for injection) (source: SHINSUN PHARMA) was weighed and heated to about 50°C under nitrogen protection, egg yolk lecithin (Lipoid E80) (source: Lipoid GmbH, Germany) and compound 2 were added under high-speed stirring, the materials were uniformly stirred, with the temperature controlled to be 55-75°C, to obtain an oil phase. Calcium sodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.82 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 2 just after same was checked to be accepted.

### Example 3

A formulation is as follows:

| | |
|---|---|
| Compound 3 | 2.5 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.3 g |
| Disodium edetate | 0.05 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Disodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.91 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 4

A pharmaceutical preparation not containing a bacteriostat was prepared according to the following formulation:

| | |
|---|---|
| Compound 3 | 2.5 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.3 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.95 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 5

A formulation is as follows:

| | |
|---|---|
| Compound 3 | 10 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.4 g |
| Sodium calcium edetate | 0.05 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Calcium sodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.87 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 6

A formulation is as follows:

| | |
|---|---|
| Compound 3 | 10 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.3 g |
| Disodium edetate | 0.05 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Disodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.69 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 7

A formulation is as follows:

| | |
|---|---|
| Compound 5 | 10 g |
| Soybean oil (for injection) | 100 g |
| Egg yolk lecithin | 12 g |
| Glycerol (for injection) | 22.5 g |
| Sodium oleate | 0.4 g |
| Sodium calcium edetate | 0.05 g |
| Sodium hydroxide | Q.s. |
| Water for injection, making up the volume to | 1,000 ml |

Soybean oil (for injection) (source: SHINSUN PHARMA) was weighed and heated to about 50°C under nitrogen protection, egg yolk lecithin (Lipoid E80) (source: Lipoid GmbH, Germany) and compound 5 were added under high-speed stirring, the materials were uniformly stirred, with the temperature controlled to be 55-75°C, to obtain an oil phase. Calcium sodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 10.05 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 5 just after same was checked to be accepted.

### Example 8

A formulation is as follows:

| | |
|---|---|
| Compound 3 | 10 g |
| Soybean oil (for injection) | 100 g |
| Medium chain triglyceride | 100 g |
| Egg yolk lecithin | 12 g |
| Glycerol (for injection) | 22.5 g |
| Sodium oleate | 0.3 g |
| Sodium pyrosulfite | 0.25 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Sodium pyrosulfite (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.99 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 9

A formulation is as follows:

| | |
|---|---|
| Compound 3 | 2.5 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.3 g |
| Disodium edetate | 0.01 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Disodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.89 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 10

A formulation is as follows:

| | |
|---|---|
| Compound 3 | 5 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.3 g |
| Benzyl alcohol | 0.01 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 3 were added under high-speed stirring, the materials were uniformly stirred, with the temperature controlled to be 55-75°C, to obtain an oil phase. Benzyl alcohol (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.49 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 11

A formulation is as follows:

| | |
|---|---|
| Compound 3 | 20 g |
| Soybean oil (for injection) | 100 g |
| Medium chain triglyceride | 100 g |
| Egg yolk lecithin | 12 g |
| Glycerol (for injection) | 22.5 g |
| Oleic acid | 0.4 g |
| Sodium pyrosulfite | 0.025 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), oleic acid (source: Lipoid GmbH, Germany), and compound 3 were added under high-speed stirring, the materials were uniformly stirred, with the temperature controlled to be 55-75°C, to obtain an oil phase. Sodium pyrosulfite (source: Hunan ER-KANG Pharmaceutical Co., Ltd) and glycerol (for injection) [source: Croda Sipo (Sichuan) Co., Ltd)] were added into an appropriate amount of water for injection, mixed, and adjusted to a pH value of 9.75 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 3 just after same was checked to be accepted.

### Example 12

A formulation is as follows:

| | |
|---|---|
| Compound 4 | 15 g |
| Soybean oil (for injection) | 200 g |
| Egg yolk lecithin | 2.4 g |
| Glycerol (for injection) | 22.5 g |
| Oleic acid | 0.3 g |
| Ethylene diamine tetraacetic acid | 0.05 g |
| Sodium hydroxide | Q.s. |
| Water for injection, making up the volume to | 1,000 ml |

Soybean oil (for injection) (source: SHINSUN PHARMA) was weighed and heated to about 50°C under nitrogen protection, egg yolk lecithin (Lipoid E80) (source: Lipoid GmbH, Germany), oleic acid (source: Lipoid GmbH, Germany), and compound 4 were added under high-speed stirring, the materials were uniformly stirred, with the temperature controlled to be 55-75°C, to obtain an oil phase. Ethylene diamine tetraacetic acid (source: Chengdu Kelong Chemical Co., Ltd) and glycerol (for injection) [source: Croda Sipo (Sichuan) Co., Ltd)] were added into an appropriate amount of water for injection, mixed, and adjusted to a pH value of 9.98 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 4 just after same was checked to be accepted.

### Example 13

A formulation is as follows:

| | |
|---|---|
| Compound 5 | 20 g |
| Soybean oil (for injection) | 100 g |
| Medium chain triglyceride | 100 g |
| Egg yolk lecithin | 12 g |
| Glycerol (for injection) | 22.5 g |
| Oleic acid | 0.4 g |
| Sodium pyrosulfite | 0.025 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), oleic acid (source: Lipoid GmbH, Germany), and compound 5 were added under high-speed stirring, the materials were uniformly stirred, with the temperature controlled to be 55-75°C, to obtain an oil phase. Sodium pyrosulfite (source: Hunan ER-KANG Pharmaceutical Co., Ltd) and glycerol (for injection) [source: Croda Sipo (Sichuan) Co., Ltd)] were added into an appropriate amount of water for injection, mixed, and adjusted to a pH value of 10.34 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 5 just after same was checked to be accepted.

### Example 14

A formulation is as follows:

| | |
|---|---|
| Compound 6 | 10 g |
| Soybean oil (for injection) | 100 g |
| Medium chain triglyceride | 100 g |
| Egg yolk lecithin | 12 g |
| Glycerol (for injection) | 22.5 g |
| Sodium oleate | 0.3 g |
| Disodium edetate | 0.01 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 6 were added under high-speed stirring, the materials were uniformly stirred, with the temperature controlled to be 55-75°C, to obtain an oil phase. Disodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.79 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 6 just after same was checked to be accepted.

### Example 15

A formulation is as follows:

| | |
|---|---|
| Compound 7 | 2.5 g |
| Olive oil | 100 g |
| Egg yolk lecithin | 12 g |
| Glycerol (for injection) | 22.5 g |
| Sodium oleate | 0.4 g |
| Sodium pyrosulfite | 0.025 g |
| Sodium hydroxide | Q.s. |
| Water for injection, making up the volume to | 1,000 ml |

Olive oil (source: Lipoid GmbH) was weighed and heated to about 50°C under nitrogen protection, egg yolk lecithin (Lipoid E80) (source: Lipoid GmbH, Germany) and compound 7 were added under high-speed stirring, the materials were uniformly stirred, with the temperature controlled to be 55-75°C, to obtain an oil phase. Sodium pyrosulfite (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 10.55 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 7 just after same was checked to be accepted.

### Example 16

A formulation is as follows:

| | |
|---|---|
| Compound 8 | 2.5 g |
| Soybean oil (for injection) | 50 g |
| Medium chain triglyceride | 50 g |
| Egg yolk lecithin | 12 g |
| Glycerol (for injection) | 22.5 g |
| Sodium oleate | 0.4 g |
| Sodium calcium edetate | 0.025 g |
| Sodium hydroxide | Q.s. |
| Water for injection, making up the volume to | 1,000 ml |

Soybean oil (for injection) (source: SHINSUN PHARMA) and a medium chain triglyceride (source: SHINSUN PHARMA) were weighed and mixed under nitrogen protection, and heated to about 50°C, egg yolk lecithin (Lipoid E80) (source: Lipoid GmbH, Germany), and compound 8 were added under high-speed stirring, the materials were uniformly stirred, with the temperature controlled to be 55-75°C, to obtain an oil phase. Calcium sodium edetate (source: Hunan ER-KANG Pharmaceutical Co., Ltd), glycerol (for injection) [source: Croda Sipo (Sichuan) Co., Ltd)] and sodium oleate (source: Lipoid GmbH, Germany) were added into an appropriate amount of water for injection, mixed, and adjusted to a pH value of 9.91 with sodium hydroxide (source: Hunan ER-KANG Pharmaceutical Co., Ltd), with the temperature controlled to be 55-75°C, to obtain an aqueous phase. The oil phase was added into the aqueous phase under high-speed stirring (by a high shear mixing emulsifier, produced by IKA) to prepare a primary emulsion, the primary emulsion was repeatedly homogenized by a high-pressure homogenizer (produced by GEA Niro), when the emulsion particles were checked to meet the requirements, the emulsion was filtered, filled under introduction of nitrogen, sterilized in a steam sterilizer, and cooled, to obtain an emulsion injection containing compound 8 just after same was checked to be accepted.

### Stability test:

The samples prepared in examples 3, 4 and 6 are reserved at 30 ± 2°C/65% ± 5% RH, 25 ± 2°C/60% ± 5% RH for a stability test. The data are shown in Table 1-3 below:

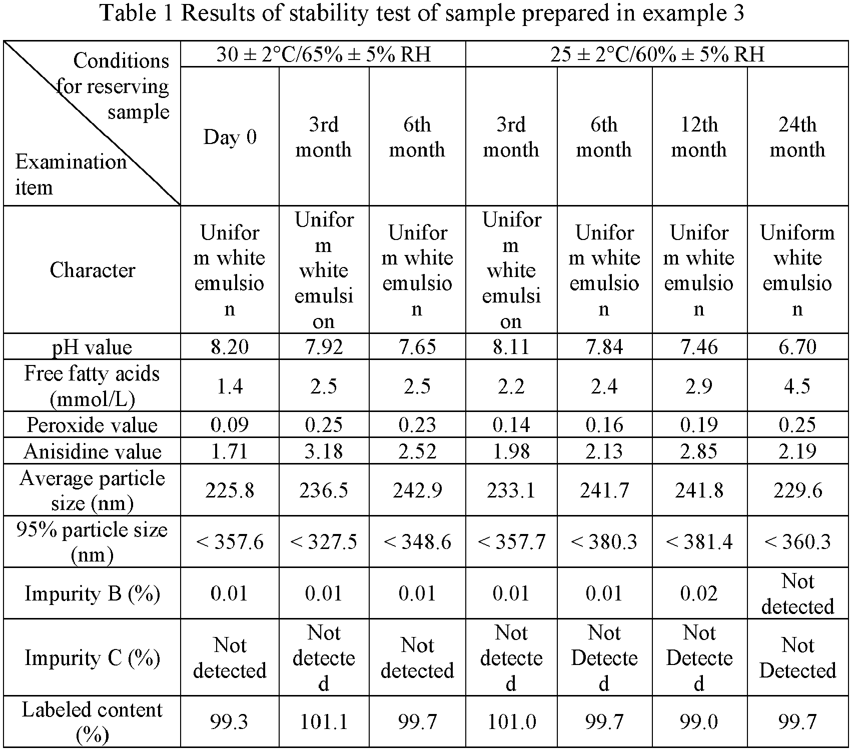

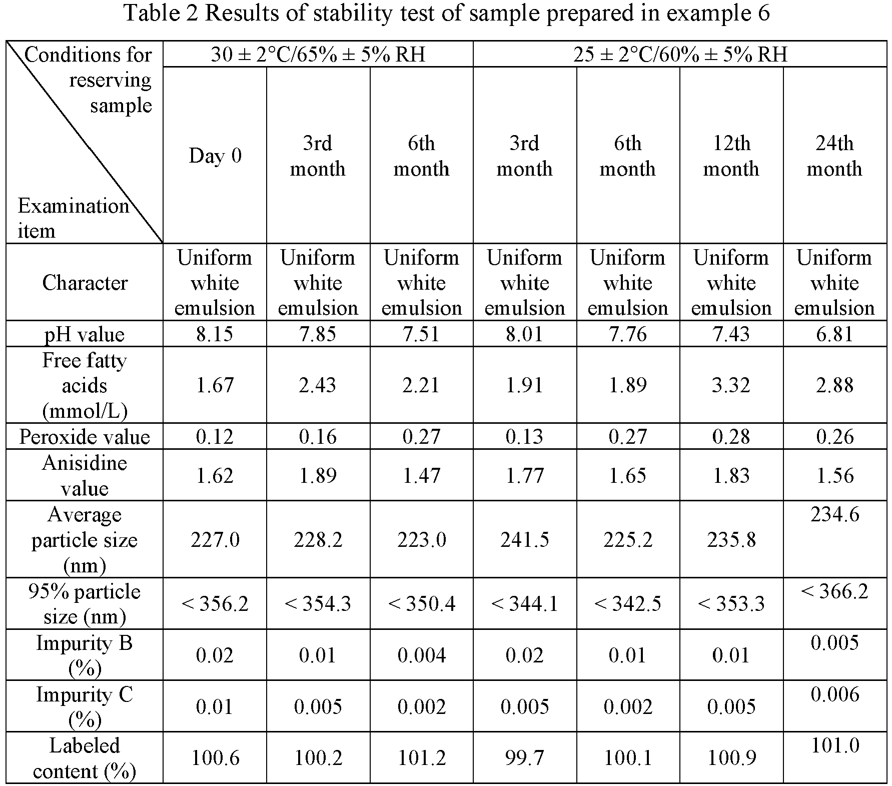

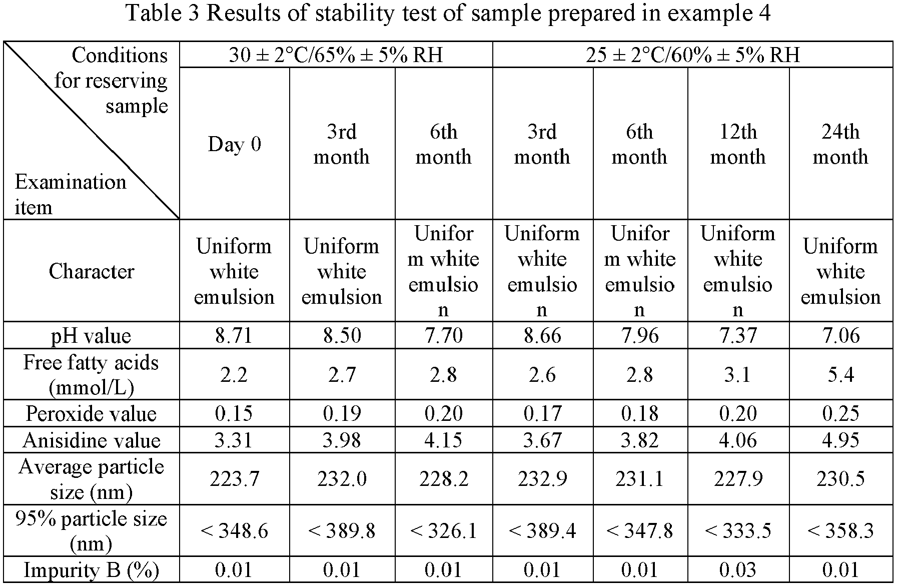

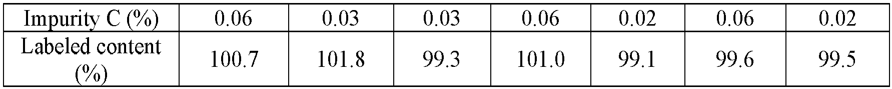

The test results showed that within the quality standard range, the anisidine value and impurity C of the samples in example 3 and example 6 are better than those of the sample in example 4, indicating that the addition of disodium edetate in the formulation could significantly improve the drug stability.

### Biological test example

### Test method:

With reference to Antimicrobial Effectiveness Testing (section 1121) of the fourth edition (2015 edition) of the Chinese Pharmacopoeia, the pharmaceutical preparation of the present invention is taken and separately inoculated with 0.1 ml (10⁵-10⁶ cfu/ml) of Staphylococcus aureus, Escherichia coli, Pseudomonas aeruginosa, and Candida albicans suspensions and an Aspergillus niger spore suspension, the materials are evenly mixed. The number of viable Staphylococcus aureus, Escherichia coli, Pseudomonas aeruginosa, Candida albicans, and Aspergillus niger is determined separately at 0 h, 2 h, 4 h, 6 h, 8 h, 10 h and 12 h according to the above verified determination method of the number of microorganisms, and the antimicrobial effectiveness is evaluated by the change of the number of microorganisms.

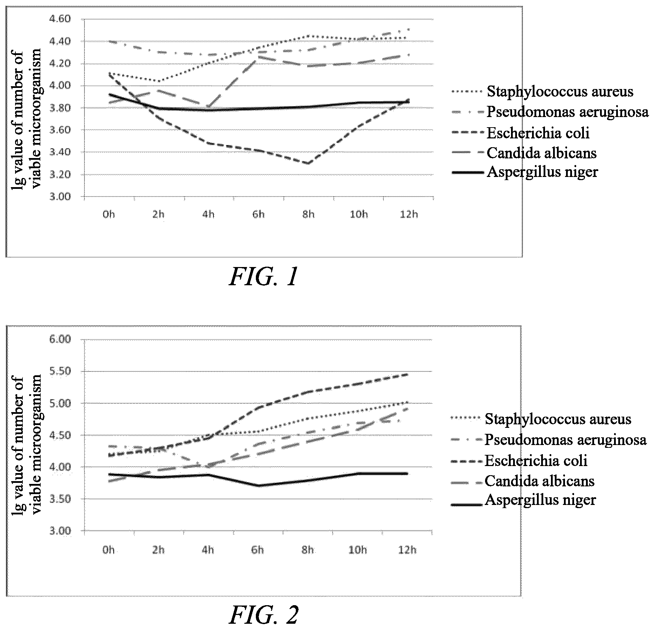

The samples of example 3 and example 4 are taken as examples. The samples prepared in example 3 and example 4 are separately taken. With reference to Antimicrobial Effectiveness Testing (section 1121) of the fourth edition (2015 edition) of the Chinese Pharmacopoeia, 10 ml of the prepared samples are separately inoculated with 0.1 ml (about 10⁵-10⁶ cfu/ml) of Staphylococcus aureus, Escherichia coli, Pseudomonas aeruginosa, and Candida albicans suspensions and an Aspergillus niger spore suspension. The number of viable Staphylococcus aureus, Escherichia coli, Pseudomonas aeruginosa, Candida albicans, and Aspergillus niger is determined separately at 0 h, 2 h, 4 h, 6 h, 8 h, 10 h and 12 h according to the verified determination method of the number of microorganisms. Growth curves of the microorganisms are shown in Fig. 1 and Fig. 2.

It can be seen from Fig. 1 and Fig. 2 that the number of viable *Staphylococcus aureus, Escherichia coli, Pseudomonas aeruginosa, Candida albicans,* and *Aspergillus niger* in the sample prepared in example 3 at 12 h is less than 0.5 lg compared with that at 0 h. Only the number of viable *Pseudomonas aeruginosa* and *Aspergillus niger* in the sample prepared in example 4 at 12 h is less than 0.5 lg compared with that at 0 h.

The concentration of the bacteriostat added in example 3 could not produce a sufficient antimicrobial effect, which is non-conforming to the antimicrobial effectiveness in the fourth edition (2015 edition) of the Chinese Pharmacopoeia. However, the above antimicrobial test results show that 0.005% disodium edetate in the prescription can actually slow down the growth of microorganisms and ensure safety of clinical medication.

### Test results:

**Table 1 Counting and conversion to logarithmic cfu/ml (lg) of test samples at different storage time after inoculation with microorganism suspensions**

| Measurement time | | 0 h | 2 h | 4 h | 6 h | 8 h | 10 h | 12 h | Compar ed with 0 h Differen t value of lg |
|---|---|---|---|---|---|---|---|---|---|
| *Staphylococ cus aureus* | Example 3 | 13,000 | 11,00 0 | 16,00 0 | 22,00 0 | 28,000 | 26,000 | 27,000 | \ |
| | Number of microorganis ms lg | 4.11 | 4.04 | 4.20 | 4.34 | 4.45 | 4.41 | 4.43 | 0.32 |
| | Example 4 | 16,000 | 18,00 0 | 32,00 0 | 36,00 0 | 58,000 | 75,000 | 102,00 0 | \ |
| | Number of microorganis ms lg | 4.20 | 4.26 | 4.51 | 4.56 | 4.76 | 4.88 | 5.01 | 0.80 |
| *Pseudomona s aeruginosa* | Example 3 | 25,000 | 20,00 0 | 19,00 0 | 20,00 0 | 21,000 | 26,000 | 32,000 | \ |
| | Number of microorganis ms lg | 4.40 | 4.30 | 4.28 | 4.30 | 4.32 | 4.41 | 4.51 | 0.11 |
| | Example 4 | 21,000 | 20,00 0 | 10,00 0 | 23,00 0 | 35,000 | 49,000 | 54,000 | \ |
| | Number of microorganis ms lg | 4.32 | 4.30 | 4.00 | 4.36 | 4.54 | 4.69 | 4.73 | 0.41 |
| *Escherichia coli* | Example 3 | 12,400 | 5,100 | 3,000 | 2,600 | 2,000 | 4,300 | 7,500 | \ |
| | Number of microorganis ms lg | 4.09 | 3.71 | 3.48 | 3.41 | 3.30 | 3.63 | 3.88 | -0.22 |
| | Example 4 | 15,000 | 20,00 0 | 28,00 0 | 86,00 0 | 150,00 0 | 200,00 0 | 280,00 0 | \ |
| | Number of microorganis ms lg | 4.18 | 4.30 | 4.45 | 4.93 | 5.18 | 5.30 | 5.45 | 1.27 |
| *Candida albicans* | Example 3 | 7,000 | 9,000 | 6,500 | 18,00 0 | 15,000 | 16,000 | 19,000 | \ |
| | Number of microorganis ms lg | 3.85 | 3.95 | 3.81 | 4.26 | 4.18 | 4.20 | 4.28 | 0.43 |
| | Example 4 | 6,000 | 9,000 | 11,00 0 | 16,00 0 | 25,000 | 39,000 | 82,000 | \ |
| | Number of microorganis ms lg | 3.78 | 3.95 | 4.04 | 4.20 | 4.40 | 4.59 | 4.91 | 1.14 |
| *Aspergillus niger* | Example 3 | 8,300 | 6,200 | 6,000 | 6,200 | 6,400 | 7,000 | 7,100 | \ |
| | Number of microorganis ms lg | 3.92 | 3.79 | 3.78 | 3.79 | 3.81 | 3.85 | 3.85 | -0.07 |
| | Example 4 | 7,700 | 6,900 | 7,600 | 5,100 | 6,100 | 7,900 | 7,900 | \ |
| | Number of microorganis ms lg | 3.89 | 3.84 | 3.88 | 3.71 | 3.79 | 3.90 | 3.90 | 0.01 |

The test results show that the growth of the contaminating microorganisms can be significantly inhibited by adding an appropriate amount of a bacteriostat ingredient such as disodium edetate, sodium calcium edetate, sodium pyrosulfite, benzyl alcohol, ethylenediaminetetraacetic acid, etc. For example, the pharmaceutical preparation of the present invention (Example 3) has an obvious inhibitory effect on the growth of the contaminating microorganisms during a service life on the basis of maintaining a good stability. The sample not containing a bacteriostat (example 4) has no inhibitory effect on the growth of *Staphylococcus aureus, Escherichia coli,* and *Candida albicans.* Therefore, the pharmaceutical preparation of the present invention reduces the potential microbial contamination risk caused by uncertain factors during the clinical use and improves safety of clinical use.

Although specific embodiments of the present invention have been described, those skilled in the art should know that the present invention can be changed and modified in many ways without departing from the range and spirit of the present invention. Therefore, the present invention is intended to cover all these changes and modifications falling within the scope of the attached claims and their equivalents.

## Claims

1. A pharmaceutical preparation, **characterized by** comprising:
(A) 0.01 w/v%-5 w/v% of an active ingredient, wherein the active ingredient is a compound of general formula (I) or a stereoisomer, a pharmaceutically acceptable salt or a prodrug thereof, 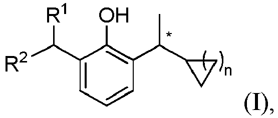 wherein R¹ and R² are each independently selected from H, C₁₋₄ alkyl or C₃₋₆ cycloalkyl; and n is selected from 1 or 2, or R¹ and R² together with the carbon atom to which they are attached form C₃₋₆ cycloalkyl; and
(B) 0.0005 w/v%-0.1 w/v% of a bacteriostat.

2. The pharmaceutical preparation according to claim 1, **characterized in that**
R¹ is selected from H, methyl, ethyl, or isopropyl; and
R² is selected from methyl, ethyl, isopropyl or cyclopropyl.

3. The pharmaceutical preparation according to claim 2, **characterized in that** the compound of general formula (I) is selected from one of the following structures:

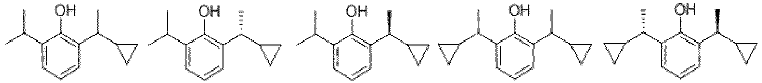

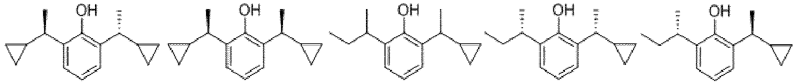

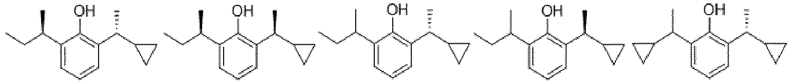

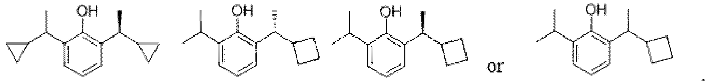

4. The pharmaceutical preparation according to claim 1, **characterized in that** the compound of general formula (I) is selected from one of the following structures:

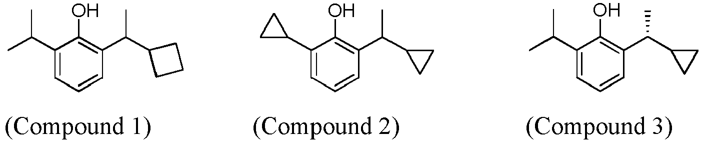

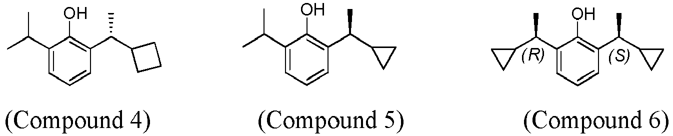

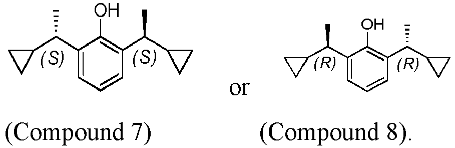

5. The pharmaceutical preparation according to claim 1, **characterized in that** the prodrug of the compound of formula (I) is selected from a compound of formula (II) below or a stereoisomer and a pharmaceutically acceptable salt:

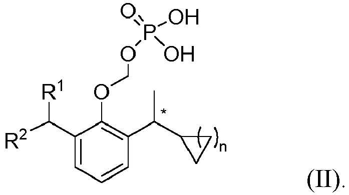

6. The pharmaceutical preparation according to any one of claims 1-5, **characterized in that** the pharmaceutical preparation is in the form of a fat emulsion, and the fat emulsion further comprises 5 w/v%-30 w/v% of an oil ingredient and 0.1 w/v%-5 w/v% of an emulsifier.

7. The pharmaceutical preparation according to claim 6, **characterized in that** the fat emulsion comprises 0.05 w/v%-2.5 w/v% of the active ingredient, 10 w/v%-25 w/v% of the oil ingredient, 0.2 w/v%-2 w/v% of the emulsifier, and 0.001 w/v%-0.03 w/v% of the bacteriostat.

8. The pharmaceutical preparation according to claim 7, **characterized in that** the fat emulsion further comprises 0-4 w/v% of an osmotic pressure regulator.

9. The pharmaceutical preparation according to claim 8, **characterized in that**
the oil ingredient is selected from any one of or a mixture of some, at any ratio, of soybean oil, olive oil, fish oil, linseed oil, medium chain triglycerides or structured triglycerides;
the emulsifier is selected from any one of or a mixture of some, at any ratio, of poloxamer, Tween-80, polyethylene glycol 15 hydroxystearate, polyoxyethylene 35 castor oil, polyoxyethylene 40 hydrogenated castor oil, egg yolk lecithin or soybean lecithin;
the bacteriostat is selected from any one of or a mixture of some, at any ratio, of sodium caprylate, benzoic acid, benzyl alcohol, sodium benzoate, methyl benzoate, sodium pyrosulfite, sodium sulfite, sodium thiosulfate, pyrogallate, ethylenediaminetetraacetic acid, disodium edetate, calcium edetate or sodium calcium edetate; and
the osmotic pressure regulator is selected from any one of or a mixture of some, at any ratio, of glycerol, saccharides or sugar alcohols.

10. The pharmaceutical preparation according to claim 8, **characterized in that** the fat emulsion further comprises a pH regulator, and the pH regulator is selected from any one or some of sodium hydroxide, potassium hydroxide, triethanolamine, hydrochloric acid, phosphoric acid, citric acid, acetic acid or malic acid; and the pharmaceutical preparation has a pH in a range of 3.0-10.0, 4.0-9.0 or 6.0-9.0.

11. The pharmaceutical preparation according to claim 10, **characterized in that** the fat emulsion further comprises 0 w/v%-2 w/v% of a stabilizer, and the stabilizer is selected from any one or a mixture of two at any ratio of oleic acid or sodium oleate.

12. The pharmaceutical preparation according to claim 11, **characterized in that** the pharmaceutical preparation comprises 0.1 w/v%-2 w/v% of the active ingredient, any one or a mixture of two at any ratio of 10 w/v%-20 w/v% of soybean oil or medium chain triglycerides, 0.2 w/v%-2 w/v% of egg yolk lecithin, 0.001 w/v%-0.03 w/v% of any one of a mixture of any two at any ratio of disodium edetate, sodium calcium edetate, sodium pyrosulfite or benzyl alcohol of any proportion, and any one or a mixture of two at any ratio of 1 w/v%-4 w/v% of glycerol and 0.01 w/v%-2 w/v% of oleic acid or sodium oleate.

13. Use of the pharmaceutical preparation according to any one of claims 1-12 in preparing a drug for inducing and maintaining anesthesia in animals or humans, promoting sedation and hypnosis in animals or humans, treating and/or preventing anxiety, depression, insomnia, nausea, vomiting, migraine, schizophrenia, convulsions, and epilepsy.
